Europäisches Patentamt

(19) European Patent Office

Office européen des brevets

(11) Veröffentlichungsnummer: **0 174 528**
**B1**

(12) **EUROPÄISCHE PATENTSCHRIFT**

(45) Veröffentlichungstag der Patentschrift:
08.03.89

(21) Anmeldenummer: **85110509.8**

(22) Anmeldetag: **21.08.85**

(51) Int. Cl.⁴: **G 01 N 33/563**, G 01 N 33/577 //
G01N33/78

(54) **Verfahren zur Bestimmung einer immunologisch bindefähigen Substanz.**

(30) Priorität: **22.08.84 DE 3430905**

(43) Veröffentlichungstag der Anmeldung:
**19.03.86 Patentblatt 86/12**

(45) Bekanntmachung des Hinweises auf die Patenterteilung:
**08.03.89 Patentblatt 89/10**

(84) Benannte Vertragsstaaten:
**AT BE CH DE FR GB IT LI LU NL SE**

(56) Entgegenhaltungen:
**DE-A-2 323 467**
**DE-B-2 743 444**
**US-A-3 940 475**
**US-A-4 235 869**
**US-A-4 361 647**
**US-A-4 423 143**

**CLINICAL CHEMISTRY, Band 28, Nr. 12, Dezember 1982, Seiten 2406-2411, Washington, US; K. MIYAI et al. "Enzyme Immunoassay of Thyroxin-Binding Globulin"**

(73) Patentinhaber: **BOEHRINGER MANNHEIM GMBH, Patentabteilung, Abt. E Sandhofer Strasse 112-132 Postfach 31 01 20, D-6800 Mannheim 31 Waldhof (DE)**

(72) Erfinder: **Lenz, Helmut, Dr. rer. nat., Waldschmidtstrasse 7, D-8132 Tutzing (DE)**
Erfinder: **Kleinhammer, Gerd, Dr. rer. nat., Heinrich- Vogl- Strasse 1, D-8132 Tutzing (DE)**

(74) Vertreter: **Weickmann, Heinrich, Dipl.- Ing., Patentanwälte Dipl.- Ing. H.Weickmann Dipl.-Phys.Dr. K.Fincke Dipl.- Ing. F.A.Weickmann Dipl.- Chem. B. Huber Dr.- Ing. H. Liska Dipl.-Phys.Dr. J. Prechtel Postfach 860820, D-8000 München 86 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Bestimmung einer immunologisch bindefähigen Substanz nach immunchemischen Methoden in heterogener Phase und ein hierfür geeignetes Reagenz.

Die Entwicklung der Immuntests hat, ausgehend vom Radioimmunverfahren (RIA), zu einer entscheidenden Verbesserung der Empfindlichkeit und Selektivität der Bestimmung von antigen wirksamen Substanzen wie Proteinen und Haptenen geführt und insbesondere in den klinisch chemischen Laboratorien große Bedeutung erlangt. Durch die Weiterentwicklung dieser Methoden wurde neben der Verbreiterung der zur Verfügung stehenden Markierungsmethoden, bei welchen neben einer radioaktiven Markierung vor allem die Enzymmarkierung (EIA) besondere Bedeutung erlangt hat, auch die Entwicklung der Umsetzungstechniken erheblich differenziert. So wurden sowohl in homogener, als auch in heterogener Phase ablaufende Tests entwickelt, obwohl bei letzteren die in der Regel erforderliche Trennung von fester und flüssiger Phase zu Verschleppungs- und Verdünnungsproblemen führen kann.

Bei den Verfahren in heterogener Phase liegt in der Regel einer der Partner einer immunologischen Bindungsreaktion an die feste Phase gebunden vor und ermöglicht nach Ablauf der entsprechenden Bindungsreaktion eine Abtrennung seines Bindungspartners aus der flüssigen Phase.

Bei derartigen, in heterogener Phase durchgeführten Verfahren wird häufig von einer Konkurrenzreaktion zwischen der zu bestimmenden Substanz und einer bestimmten Menge eines markierten Derivats der zu bestimmenden Substanz Gebrauch gemacht. Das markierte Derivat wird dabei in einer Menge an die feste Phase fixiert, die umso geringer ist, je größer die um die Bindungsstellen konkurrierende Menge an zu bestimmendem Reaktionspartner ist. Die Markierung in dem um die Bindungsstelle konkurrierenden Derivat kann dabei radioaktiv oder optisch aktiv sein, häufig wird jedoch eine Markierung durch ein bestimmbares Enzym bevorzugt. Diese Bevorzugung beruht darauf, daß eine Bestimmung dieses Markierungsenzyms ohne großen apparativen Aufwand, wie er bei radioaktiver Markierung und bei vielen optischen Markierungen erforderlich ist, erfolgen kann und derartige Enzymbestimmungsmethoden in großem Umfange bereits automatisiert wurden.

Ein wesentlicher Nachteil derartiger Verfahren unter Verwendung eines bestimmbaren Enzyms als Marker besteht jedoch darin, daß es hierbei erforderlich wird, das Enzym kovalent zu verknüpfen, was eine Reihe von Nachteilen aufweist. So wird bei der kovalenten Umsetzung eines Enzyms regelmäßig ein erheblicher Teil seiner Aktivität verlorengehen, was beträchtliche Kosten verursacht. Zum anderen sind derartige Bindungsmethoden heikel und häufig schwierig durchzuführen. Besonders problematisch sind in der Regel dabei die Aufarbeitungsbedingungen, die in der Regel eine affinitätschromatographische Reinigung umfassen. Unter den für die Elution erforderlichen Bedingungen wird aber wiederum ein Teil der Enzymaktivität zerstört, beispielsweise durch niedrigen pH-Wert.

Der Erfindung liegt daher die Aufgabe zugrunde, die geschilderten Nachteile der bekannten Verfahren zu beseitigen und ein immunologisches Bestimmungsverfahren in heterogener Phase zu schaffen, bei welchem die Vorteile einer Enzymmarkierung mit den Vorteilen der vergleichsweise unempfindlichen radioaktiven oder optischen Markierung vereinigt werden.

Diese Aufgabe wird erfindungsgemäß gelöst durch ein Verfahren zur Bestimmung einer immunologisch bindefähigen Substanz, vorzugsweise eines Haptens, nach immunchemischen Methoden in heterogener Phase, welches dadurch gekennzeichnet ist, daß man

a)    eine zu bestimmende Substanz enthaltende Probelösung mit

b)    einem festphasengebundenen Antikörper gegen die zu bestimmende bindefähige Substanz,

c)    einem Konjugat aus der zu bestimmenden bindefähigen Substanz und einem Fab-Fragment eines gegen ein bestimmbares Enzym gerichteten Antikörpers in einer bestimmten Menge und

d)    dem Enzym, welchem dem im Konjugat von c) enthaltenen Antikörper entspricht,

inkubiert, die Phasen abtrennt und die Enzymaktivität in einer der beiden Phasen mißt.

Erfindungswesentlich ist die Verwendung eines nativen Enzyms als Markierung in Kombination mit einem Konjugat aus einem Fab-Fragment des entsprechenden Antienzym-Antikörpers mit der zu bestimmenden bindefähigen Substanz. Dieses Konjugat konkurriert mit der in der zu untersuchenden Lösung enthaltenen und zu bestimmenden freien immunologisch bindefähigen Substanz um den festphasengebundenen Antikörper.

Als festphasengebundener Antikörper gegen die zu bestimmende bindefähige Substanz kommen sowohl vollständige, polyklonale oder monoklonale Antikörper als auch Fragmente derselben in betracht. Die feste Phase, an welche diese Antikörper gebunden ist, kann in kompakter Form oder teilchenförmig vorliegen. Als Trägermaterial kommen alle Substanzen in betracht, an welche biologisch aktive Proteine unter Erhalt ihrer biologischen Aktivität gebunden werden können. Derartige Substanzen sind dem Fachmann in großer Zahl bekannt. Am häufigsten handelt es sich um Glas, Kunststoffe oder synthetische oder natürliche Polymere wie z. B. Cellulose, Dextrane, Styrolpolymerisate und dergleichen. Auch Ionenaustauscher und unspezifische Adsorber können verwendet werden. Die Bindung des Antikörpers an das Trägermaterial erfolgt durch Adsorption, kovalente Bindung, über ionische Bindungen, durch Polymerisation und dergleichen. Alle diese Verfahren sind dem Fachmann bekannt. Handelt es sich um einen polyvalenten Antikörper, so kann dieser auch durch immunologische Präzipitation auf dem Trägermaterial fixiert sein.

Als weiteres Reagenz verwendet die Erfindung ein Konjugat aus der zu bestimmenden bindefähigen Substanz, vorzugsweise einem Hapten, und einem Fab-Fragment eines Antikörpers, der gegen das im Bestimmungsverfahren zu verwendende bestimmbare Enzym gerichtet ist. Diese Antienzym-Fab-Fragmente können entweder monoklonal sein, also in der Zellkultur gewonnen werden, oder auf herkömmlichem Wege durch Immunisierung von Tieren gegen das Bestimmungsenzym, Gewinnung der gebildeten Antikörper, beispielsweise über Affinitätschromatographie und Spaltung, zur Gewinnung der Fab-Fragmente gewonnen werden. Hierfür geeignete Methoden sind beispielsweise in "Alan Johnstone und Robin Thorpe, Immunochemistry in Practice Blackwell Scientific Publications, 1982, Oxford" beschrieben.

Auch die Verknüpfung so gewonnener Antienzym-Fab-Fragmente mit der zu bestimmenden Substanz, beispielsweise einem Hapten, erfolgt nach hierfür bekannten Methoden, beispielsweise unter Verwendung von difunktionellen Brückenbildnern wie aktiven Estern, Dialdehyden und dergleichen.

Als Enzym kann im Rahmen der Erfindung jedes bestimmbare Enzym verwendet werden. Vorzugsweise verwendet man die im Rahmen der Enzymimmunverfahren bewährten Enzyme, die sich in der Regel durch Stabilität, leichte Zugänglichkeit und einfache Bestimmbarkeit auszeichnen. Typische Beispiele für derartige Enzyme sind Peroxidase, alkalische Phosphatase, β-Galactosidase, Glucoamylase, Glucoseoxidase, Acetylcholinesterase, Katalase, Lysozym, Malatdehydrogenase, Glucose-6-phosphat-dehydrogenase und β-Amylase. Besonders bevorzugt werden Peroxidase und β-Galactosidase.

Es ist zwar bereits ein Verfahren bekannt (Z. med. Labor.-Diagn. 25 (1984), 196 ff), bei welchem Antikörper-Chimären verwendet werden, die durch Verknüpfung von kompletten Antikörpern mit unterschiedlicher Antigenspezifität entstehen. Dabei werden auch solche Antikörper-Chimären in betracht gezogen, die aus einem antigenspezifischen und einem enzymspezifischen Antikörper bestehen. Bei Verwendung einer derartigen Antikörper-Chimäre wäre es jedoch notwendig, die Messung eines Markierungsenzyms an der festen Phase durchzuführen, da eine derartige Chimäre das Enzym präzipitiert und damit an der Oberfläche der festen Phase bindet, falls im heterogenen System gearbeitet wird.

Demgegenüber schafft die Erfindung die Möglichkeit, das Enzym auch in der flüssigen Phase quantitativ zu bestimmen, indem man das Enzym in einer bekannten Menge einsetzt. Hierdurch wird z. B. die Durchführung des gesamten Verfahrens in äußerst einfacher Weise in Teströhrchen möglich. So kann man beispielsweise den verwendeten kompletten Antikörper adsorptiv an die innere Oberfläche eines Kunststoffreagenzglases oder in den Vertiefungen einer Titerplatte aus Kunststoff wie Polystyrol, binden und dann eine Lösung zusetzen, die das Konjugat gemäß c), das Enzym gemäß d) und die Probelösung a) enthält. Nach Beendigung der Inkubation wird die flüssige Phase entnommen und die darin enthaltene Enzymaktivität als Maß für die Menge der zu bestimmenden Substanz gemessen. Gemäß einer anderen Ausführungsform kann man als festphasengebundenen Antikörper b) einen durch ionische Bindung, Immunpräzipitation oder kovalent an ein Vlies gebundenen Antikörper verwenden, dem die oben angegebenen Reaktionspartner zugesetzt und nach Ablauf der Inkubation durch physikalische Methoden, beispielsweise durch Kapillarkraft oder Zentrifugalkraft wieder abgezogen werden.

Als Antienzym-Antikörper-Fab-Fragmente werden vorzugsweise solche verwendet, die die Aktivität des Enzyms nicht beeinflussen, also weder aktivieren noch hemmen. Jedoch können auch teilweise hemmende und aktivierende Fragmente verwendet werden.

Bei der Durchführung des erfindungsgemäßen Verfahrens kann das Konjugat gemäß c) und das Enzym gemäß d) dem Bestimmungsansatz getrennt zugesetzt werden. Alternativ ist es aber auch möglich, die Reaktanten c) und d) auch als Vormischung einzusetzen.

Ein weiterer Gegenstand der Erfindung ist ein Reagenz zur Bestimmung einer immunologisch bindefähigen Substanz, welches dadurch gekennzeichnet ist, daß es einen festphasengebundenen Antikörper gegen die zu bestimmende bindefähige Substanz, ein Konjugat aus der zu bestimmenden bindefähigen Substanz und einem Fab-Fragment eines gegen ein bestimmbares Enzym gerichteten Antikörpers, ein bestimmbares Enzym, welches mit dem im Konjugat enthaltenen Fab-Fragment immunologisch bindet und Puffer enthält.

Vorzugsweise enthält das erfindungsgemäße Enzym zusätzlich ein System zur Bestimmung der Enzymaktivität.

Hinsichtlich des festphasengebundenen Antikörpers, des Konjugats und des Enzyms gelten die oben, in Bezug auf das Verfahren gemachten Ausführungen hier in gleicher Weise. Der gewählte Puffer wird in erster Linie durch das jeweils verwendete Enzym bedingt und soll so gewählt werden, daß hinsichtlich Konzentration und pH-Wert für die Aktivität des Enzyms günstige Bedingungen vorliegen. Diese Bedingungen und die geeigneten Puffer und Pufferkonzentrationen sind dem Fachmann bekannt und brauchen hier nicht näher erläutert zu werden.

Das System zur Bestimmung der Enzymaktivität richtet sich wiederum nach dem verwendeten Enzym. Es können hierfür die handelsüblichen Enzymbestimmungsreagenzien verwendet werden, die in großer Anzahl und Vielfalt zur Verfügung stehen. Bei Verwendung von Peroxidase nutzt man in der Regel die durch dieses Enzym bewirkte Oxidation in Gegenwart von $H_2O_2$ aus und setzt ein entsprechendes Bestimmungssystem ein.

Wird als Enzym β-Galactosidase verwendet, so besteht das System zur Bestimmung seiner Aktivität vorzugsweise aus einem Galactosid mit einer optisch leicht bestimmbaren Gruppe wie beispielsweise Nitrophenylgalactosid, Dinitrophenylgalactosid, Aminophenylgalactosid und dergleichen.

Als feste Phase enthält das erfindungsgemäße Reagenz vorzugsweise ein Kunststoffröhrchen oder eine Kunststoffplatte mit an der Oberfläche adsorptiv gebundenem Antikörper. Ein anderer bevorzugter festphasengebundener Antikörper besteht aus einem Vlies, beispielsweise Papier, an welches der Antikörper nach einer der oben beschriebenen Methoden fixiert vorliegt.

Durch die Erfindung wird erreicht, daß bei einer immunchemischen Bestimmung in heterogener Phase mit Verwendung einer Enzymmarkierung ein natives Enzym eingesetzt werden kann und keine vorherige Bindung an eine andere Substanz nötig ist. Bei den bisher benötigten Hapten/Antigen-Enzym-Konjugaten wird im Rahmen der affinitätschromatographischen Reinigung in der Regel die Enzymaktivität durch die Elutionsbedingungen weitgehend zerstört. Bei der Erfindung können dagegen die benötigten Konjugate, da sie kein Enzym enthalten, durch haptenspezifische oder antigenspezifische affinitätschromatographische Reinigung auf 100 % Immunreaktivität gebracht werden.

Die folgenden Beispiele erläutern die Erfindung weiter.

## Beispiel 1

Bestimmung von T4 (Tyroxin)

Reagenzien:

| | | |
|---|---|---|
| β-Galactosidase aus E. coli | 5 mg/ml | |
| | 30 U/mg | |
| T4-Konjugat mit Anti-β Galactosidase-Antikörper Fab-Fragment, verbunden unter Verwendung von N-t-Butoxy carbonyl-Tyroxyl-o-succinimid | 0,88 mg/ml | $\hat{=}$ 164 mU/ml |
| Anti-T4-Antikörper, adsorptiv | | |

an Polystyrolröhrcheninnenwand gebunden

Inkubationspuffer
$MgCl_2$
$NaH_2PO_4$.

Herstellung der Lösungen:
1) Inkubationspuffer wird in 100 ml bidestilliertem Wasser gelöst.
2) 100 µl Konjugatlösung werden in 25 ml Inkubationspuffer gelöst.
3) Substratpuffer: 2 mM $MgCl_2$, 100 mM $NaH_2PO_4$, pH 7,2.
4) Substrat: 100 mg Nitrophenylgalactosid in 20 ml Substratpuffer
5) 30 µl β-Galactosidase in 3 ml 2,2 M Ammoniumsulfat, pH 6 lösen = 1,5 U/ml.
6) 0,5 ml Lösung 5 + 20 ml Substratpuffer mischen = 37,5 mU/Test.

## Durchführung der Bestimmung:

In die Kunststoffröhrchen werden 100 µl T4-Standard und 1 ml Lösung 2 pipettiert. Dann wird eine Stunde zur Inkubation stehengelassen, danach die Flüssigkeit ausgesaugt, das Röhrchen mit Leitungswasser gespült und erneut ausgesaugt. Anschließend wird 1 ml Lösung 6 zugesetzt, wieder eine Stunde stehengelassen und ausgesaugt, gespült und nochmals ausgesaugt, wie oben beschrieben. Danach wird 1 ml Substratlösung 4 zugegeben und nach 30 Minuten die Extinktion bei 405 nm gegen Substratpuffer gemessen.

## Patentansprüche

1. Verfahren zur Bestimmung einer immunologisch bindefähigen Substanz, vorzugsweise eines Haptens, nach immunchemischen Methoden in heterogener Phase, dadurch gekennzeichnet, daß man

a) eine die zu bestimmende Substanz einhaltende Probelösung mit
b) einem festphasengebundenen Antikörper gegen die zu bestimmende bindefähige Substanz,
c) einem Konjugat aus der zu bestimmenden bindefähigen Substanz und einem Fab-Fragment eines gegen ein bestimmbares Enzym gerichteten Antikörpers in einer bestimmten Menge und
d) dem Enzym, welches dem im Konjugat von c) enthaltenen Antikörper entspricht,

inkubiert, die Phasen trennt und die Enzymaktivität in einer der beiden Phasen mißt.
2. Verfahren nach Anspruch 1, dadurch gekennzeichnet, daß man eine bekannte Menge des bestimmbaren Enzyms gemäß d) einsetzt und die Enzymaktivität in der flüssigen Phase mißt.

3. Verfahren nach Anspruch 1 oder 2, <u>dadurch gekennzeichnet</u>, daß der Antikörper gegen die zu bestimmende bindefähige Substanz gemäß b) an die feste Phase adsorptiv, kovalent, durch Einpolymerisation oder durch Vernetzung gebunden ist.

4. Verfahren nach einem der vorhergehenden Ansprüche, <u>dadurch gekennzeichnet</u>, daß man als bestimmbares Enzym d) β-Galactosidase oder Peroxidase verwendet.

5. Verfahren nach einem der vorhergehenden Ansprüche, <u>dadurch gekennzeichnet</u>, daß man im Konjugat c) ein monoklonales Fab-Fragment verwendet.

6. Reagenz zur Bestimmung einer immunologisch bindefähigen Substanz, <u>dadurch gekennzeichnet</u>, daß es einen festphasengebundenen Antikörper gegen die zu bestimmende bindefähige Substanz, ein Konjugat aus der zu bestimmenden bindefähigen Substanz und einem Fab-Fragment eines gegen ein bestimmbares Enzym gerichteten Antikörpers, ein bestimmbares Enzym, welches mit dem im Konjugat enthaltenen Fab-Fragment immunologisch bindet und Puffer enthält.

7. Reagenz nach Anspruch 6, <u>dadurch gekennzeichnet</u>, daß es zusätzlich ein System zur Bestimmung der Enzymaktivität enthält.

8. Reagenz nach Anspruch 6 oder 7, <u>dadurch gekennzeichnet</u>, daß die feste Phase aus einem Kunststoffröhrchen besteht, an dessen Oberfläche der Antikörper adsorptiv gebunden ist.

9. Reagenz nach Anspruch 6 oder 7, <u>dadurch gekennzeichnet</u>, daß die feste Phase aus einem Vlies besteht, an welches der Antikörper kovalent oder durch Antigen-Antikörper-Komplexbildung gebunden ist.

10. Reagenz nach einem der vorhergehenden Ansprüche 6 bis 9, <u>dadurch gekennzeichnet</u>, daß es als bestimmbares Enzym β-Galactosidase oder Peroxidase enthält.

separates the phases and measures the enzyme in one of the two phases.

2. Process according to claim 1, characterised in that one uses a known amount of the determinable enzyme according to d) and measures the enzyme activity in the liquid phase.

3. Process according to claim 1 or 2, characterised in that the antibody against the bindable substance to be determined according to b) is bound to the solid phase adsorptively, covalently, by polymerising in or by cross-linking.

4. Process according to one of the preceding claims, characterised in that, as determinable enzyme d), one uses β-galactosidase or peroxidase.

5. Process according to one of the preceding claims, characterised in that one uses a monoclonal Fab fragment in the conjugate c).

6. Reagent for the determination of an immunologically-bindable substance, characterised in that it contains a solid phase-bound antibody against the bindable substance to be determined, a conjugate of the bindable substance to be determined and a Fab fragment of an antibody directed against a determinable enzyme, a determinable enzyme which binds immunologically with the Fab fragment contained in the conjugate and buffer.

7. Reagent according to claim 6, characterised in that it additionally contains a system for the determination of the enzyme activity.

8. Reagent according to claim 6 or 7, characterised in that the solid phase consists of a synthetic material test tube on the surface of which the antibody is adsorptively bound.

9. Reagent according to claim 6 or 7, characterised in that the solid phase consists of a fleece to which the antibody is bound covalently or by antigen-antibody complex formation.

10. Reagent according to one of the preceding claims 6 to 9, characterised in that it contains β-galactosidase or peroxidase as determinable enzyme.

## Claims

1. Process for the determination of an immunologically-bindable substance, preferably of a hapten, by immunochemical methods in heterogeneous phase, characterised in that one incubates

a) a sample solution containing the substance to be determined with
b) a solid phase-bound antibody against the bindable substance to be determined,
c) a conjugate of the bindable substance to be determined and a Fab fragment of an antibody directed against a determinable enzyme in a definite amount and
d) the enzyme which corresponds to the antibody contained in the conjugate c),

## Revendications

1. Procédé pour la détermination d'une substance capable de se lier immunologiquement, de préférence d'un haptène, par des méthodes immunolochimiques en phase hétérogène, caractérisé en ce qu'on fait incuber

a) une solution d'échantillon contenant la substance à déterminer, avec,
b) un anticorps lié à une phase solide contre la substance capable de se lier à déterminer
c) un conjugué de la substance capable de se lier à déterminer et d'un fragment Fab d'un anticorps dirigé contre une enzyme dosable dans une quantité déterminée, et
d) l'enzyme qui correspond à l'anticorps contenu dans le conjugué de (c), on sépare les phases et on mesure l'activité enzymatique dans une des deux phases.

2. Procédé suivant la revendication 1, caractérisé en ce qu'on utilise une quantité connue de l'enzyme à déterminer selon d) et en ce qu'on mesure l'activité de l'enzyme dans la phase liquide.

3. Procédé suivant les revendications 1 ou 2, caractérisé en ce que l'anticorps contre la substance capable de se lier à déterminer selon b) est lié à la phase solide par adsorption, par covalence, par polymérisation ou par réticulation.

4. Procédé suivant l'une des revendications précédentes, caractérisé en ce qu'on utilise comme enzyme dosable d) de la β-galactosidase ou de la peroxydase.

5. Procédé suivant l'une des revendications précédentes, caractérise en ce qu'on utilise dans le conjugué d) un fragment Fab monoclonal.

6. Réactif pour la détermination d'une substance capable de se lier immunologiquement, caractérisé en ce qu'il contient un anticorps lié à une phase solide contre la substance capable de se lier à déterminer un conjugué de la substance capable de se lier à déterminer et d'un fragment Fab d'un anticorps dirigé contre une enzyme dosable, une enzyme dosable qui se lie au fragment Fab contenu dans le conjugué et un tampon.

7. Réactif suivant la revendication 6, caractérisé en ce qu'il contient en outre un système pour la détermination de l'activité de l'enzyme.

8. Réactif suivant les revendications 6 ou 7, caractérisé en ce que la phase solide se compose d'un petit tube de matière plastique à la surface duquel l'anticorps est lié par adsorption.

9. Réactif suivant les revendications 6 ou 7, caractérisé en ce que la phase solide se compose d'une nappe fibreuse à laquelle l'anticorps est lié par covalence ou par formation de complexe antigène-anticorps.

10. Réactif suivant l'une des revendications 6 à 9 qui précèdent, caractérisé en ce qu'il contient comme enzyme dosable de la β-galactosidase ou de la peroxydase.